# EUROPEAN PATENT SPECIFICATION

(11) **EP 0 914 454 B1**
(45) Date of publication and mention of the grant of the patent: **09.03.2005**
(21) Application number: 97929831.2
(22) Date of filing: 10.06.1997
(51) Int. Cl.: C12N 15/82, C12N 1/20, A01H 5/00

(54) **A SYNTHETIC PLANT CORE PROMOTER AND UPSTREAM REGULATORY ELEMENT**
EIN SYNTHETISCHER, PFLANZLICHER KERNPROMOTOR UND STROMAUFWAERTS GELEGENES, REGULATORISCHES ELEMENT
PROMOTEUR DE COEUR DE PLANTE SYNTHETIQUE ET ELEMENT REGULATEUR EN AMONT

(30) Priority: 11.06.1996 US 661601
(43) Date of publication of application: 12.05.1999
(73) Proprietor: PIONEER HI-BRED INTERNATIONAL, INC., Des Moines, Iowa 50309 (US)
(72) Inventor: BRUCE, Wesley, B., Urbandale, IA 50322 (US); SIMS, Lynne, E., Polk City, IA 50226 (US)
(74) Representative: Chapman, Paul William
(86) International application number: PCT/US1997/009849
(87) International publication number: WO 1997/047756

(56) References cited:
- EP-A- 0 278 659
- EP-A- 0 342 926
- EP-A- 0 459 643
- WO-A-94/01571
- WO-A-95/14098
- US-A- 5 097 025
- US-A- 5 106 739
- US-A- 5 573 932

## Description

### FIELD OF THE INVENTION

This invention relates generally to the field of plant molecular biology and in particular to a synthetic upstream element and the combined arrangement of such an element with a promoter within a promoter region such that gene expression is enhanced. The invention enables the enhanced expression of desired structural genes in both monocotyledonous and dicotyledonous plants.

### BACKGROUND OF THE INVENTION

Gene expression encompasses a number of steps originating from the DNA template ultimately to the final protein or protein product. Control and regulation of gene. expression can occur through numerous mechanisms. The initiation of transcription of a gene is generally thought of as the predominant control of gene expression. The transcriptional controls (or promoters) are generally relegated to relatively short sequences embedded in the 5'-flanking or upstream region of the transcribed gene. There are DNA sequences which affect gene expression in response to environmental stimuli, nutrient availability, or adverse conditions including heat shock, anaerobiosis or the presence of heavy metals. There are also DNA sequences which control gene expression during development or in a tissue, or organ specific fashion.

Promoters contain the signals for RNA polymerase to begin transcription so that protein synthesis can proceed. DNA binding, nuclear proteins interact specifically with these cognate promoter DNA sequences to promote the formation of the transcriptional complex and eventually initiate the gene expression process.

One of the most common sequence motifs present in the promoters of genes transcribed by eukaryotic RNA polymerase II (poIII) system is the "TATA" element which resides upstream of the start of transcription. Eukaryotic promoters are complex and are comprised of components which include a TATA box consensus sequence at about 35 base pairs 5' relative to the transcription start site or cap site which is defined as +1. The TATA motif is the site where the TATA-binding-protein (TBP) as part of a complex of several polypeptides (TFIID complex) binds and productively interacts (directly or indirectly) with factors bound to other sequence elements of the promoter. This TFIID complex in turn recruits the RNA polymerase II complex to be positioned for the start of transcription generally 25 to 30 base pairs downstream of the TATA element and promotes elongation thus producing RNA molecules. The sequences around the start of transcription (designated INR) of some polII genes seem to provide an alternate binding site for factors that also recruit members of the TFIID complex and thus "activate" transcription. These INR sequences are particularly relevant in promoters that lack functional TATA elements providing the core promoter binding sites for eventual transcription. It has been proposed that promoters containing both a functional TATA and INR motif are the most efficient in transcriptional activity. (Zenzie-Gregory et al, 1992. J. Biol. Chem. 267:2823-2830).

In most instances sequence elements other than the TATA motif are required for accurate transcription. Such elements are often located upstream of the TATA motif and a subset may have homology to the consensus sequence CCAAT.

Other DNA sequences have been found to elevate the overall level of expression of the nearby genes. One of the more common elements that have been described reside far upstream from the initiation site and seem to exhibit position and orientation independent characteristics. These far upstream elements have been designated enhancers.

One of the less common elements by virtue of their specificities are sequences that interact with specific DNA binding factors. These sequence motifs are collectively known as upstream elements which are usually position and orientation dependent.

Many upstream elements have been identified in a number of plant promoters based initially cn function and secondarily on sequence homologies. These promoter upstream elements range widely in type of control: from environmental responses like temperature, moisture, wounding, etc., developmental cues, (germination, seed maturation, flowering, etc.) to spatial information (tissue specificity). These elements also seem to exhibit modularity in that they may be exchanged with other elements while maintaining their characteristic control over gene expression.

Promoters are usually positioned 5' or upstream relative to the start of the coding region of the corresponding gene, and the entire region containing all the ancillary elements affecting regulation or absolute levels of transcription may be comprised of less than 100 base pairs or as much as 1 kilobase pair.

A number of promoters which are active in plant cells have been described in the literature. These include nopaline synthase (NOS) and octopine synthase (OCS) promoters (which are carried on tumor inducing plasmids of *Agrobact*e*rium tumefaciens*). The cauliflower mosaic virus (CaMV) 19S and 35S promoters, the light-inducible promoter from the small subunit of ribulose bisphosphate carboxylase (ssRUBICSO), a very abundant plant polypeptide), and the sucrose synthase promoter are also included. Promoters for use in plants are also disclosed in EP-A-O 459 643; WO-A-95 14038; and EP-A-O 342 926. All of these promoters have been used to create various types of DNA constructs which have been expressed in plants. (See for example PCT publication WO84/02913 Rogers *et al*.)

Two promoters that have been widely used in plant cell transformations are those of the genes encoding alcohol dehydrogenase, AdhI and AdhII. Both genes are induced after the onset of anaerobiosis. Maize AdhI has been cloned and sequenced as has been AdhII. Formation of an AdhI chimeric gene, Adh-CAT comprising the AdhI promoter links to the chloramphenicol acetyltransferase (CAT) coding sequences and nopaline synthase (NOS) 3' signal caused CAT expression at approximately 4-fold higher levels at low oxygen concentrations than under control conditions. Sequence elements necessary for anaerobic induction of the ADH-CAT chimeric have also been identified. The existence of anaerobic regulatory element (ARE) between positions -140 and -99 of the maize AdhI promoter composed of at least two sequence elements positions -133 to -124 and positions -113 to 99 both of which have found to be necessary and are sufficient for low oxygen expression of ADH-CAT gene activity. The Adh promoter however responds to anaerobiosis and is not a constitutive promoter drastically limiting its effectiveness.

Another commonly used promoter is the 35S promoter of Cauliflower Mosaic Virus. The (CaMV) 35S promoter is a dicot virus promoter however it directs expression of genes introduced into protoplasts of both dicots and monocots. The 35S promoter is a very strong promoter and this accounts for its widespread use for high level expression of traits in transgenic plants. The CaMV35S promoter however has also demonstrated relatively low activity in several agriculturally significant graminaceous plants such as wheat. While these promoters all give high expression in dicots, none give high levels of expression in monocots. A need exists for a synthetic promoters and other elements that induce expression in transformed monocot protoplast cells.

It is a primary object of this invention to provide synthetic regulatory elements that enhance expression of introduced genes in plant cells and plant tissues.

It is another object of this invention to provide synthetic upstream enhancer elements that can further stimulate the activity of any promoter.

It is yet another object of this invention to provide plants, plant cells and plant tissues containing the upstream element of the invention.

It is yet another object of the invention to provide vehicles for transformation of plant cells including viral or plasmid vectors and expression cassettes incorporating the upstream elements of the invention.

It is yet another object of the invention to provide bacterial cells comprising such vectors for maintenance, replication, and plant transformation.

Other objects of the invention will become apparent from the description of the invention which follows.

### SUMMARY OF THE INVENTION

In a first aspect, the present invention provides a synthetic upstream element having a sequence of SEQ ID NO:2.

In a second aspect, the invention provides an expression cassette comprising:
a promoter;
a structural gene operatively linked to said promoter sequence;
a polyadenylation signal; and
a synthetic upstream element comprising SEQ ID NO:2 operatively linked to said promoter so that expression is enhanced.

The promoter may be a synthetic promoter comprising a TATA motif; a transcription start site, and a region between the TATA motif and the transcription start site that is at least 64% GC rich. The promoter may be SEQ ID NO:1 or SEQ ID NO:10.

In a third aspect, the invention provides a nucleic acid vector comprising the expression cassette of the second aspect.

In a fourth aspect, the invention provides a nucleic acid vector comprising:
(i) a promoter operatively linked to a structural gene, wherein the promoter is a synthetic DNA plant promoter sequence which comprises:
   a TATA motif;
   a transcription start site; and
   a region between the TATA motif and the start site that is at least 64% GC-rich; and
(ii) an upstream element operatively linked to said promoter comprising the sequence of SEQ ID NO:2.

In the vector of the fourth aspect, the promoter sequence may be SEQ ID NO:10 or SEQ ID NO:1. The vector of the invention may be a cloning vector or an expression vector. In one embodiment, the vector further comprises a marker gene for selection of transformed cells, such as an antibiotic resistance gene. The vector may further comprise a polyadenylation signal.

The invention also provides a prokaryotic or eukaryotic host cell transformed with the nucleic acid vector of the fourth aspect, and a transgenic plant comprising a plant cell or ancestor thereof which has been transformed with the vector of the fourth aspect, as well as transformed seed of such a transgenic plant.

The invention provides an upstream element that will enable those of skill in the art to obtain reliable high levels of expression of introduced structural genes in target cells. The element may be used in combination with a synthetic core promoter which comprises a TATA box and a start of transcription. Further, the "TATA to start" region is 64% or greater GC rich. One embodiment of the core promoter is shown in SEQ ID NO:1. The upstream element can be used in combination with various plant core promoter sequences. In a preferred embodiment the core promoter and upstream element are used together to obtain ten-fold higher expression of an introduced marker gene in monocot transgenic plants then is obtained with the maize ubiquitin 1 promoter.

The core promoter comprises a TATA motif and a GC rich "TATA to start of transcription" region (64% or greater GC content that is traditionally characteristic of animal promoters. The sequence is placed 5' of a structural gene and will promote constitutive expression which is non-tissue specific in transgenic plant cells.

The invention also comprises an expression cassette comprising the upstream element, the synthetic core promoter, a structural gene, the expression of which is desired in plant cells, and a polyadenylation or stop signal. The expression cassette can be encompassed in plasmid or viral vectors for transformation of plant protoplast cells.

The invention also encompasses transformed bacterial cells for maintenance and replication of the vector, as well as transformed monocot or dicot cells and ultimately transgenic plants.

The invention also comprises an expression cassette comprising the synthetic upstream element of the invention, 5' to a plant inducible promoter which is 5' to a structural gene. This expression cassette can be embodied in vectors and plasmids as earlier described.

In a preferred embodiment the synthetic upstream element is used in combination with the synthetic core promoter sequence to achieve nontissue specific constitutive expression of the gene product which is a ten-fold enhancement of the maize ubi1 promoter.

### DESCRIPTION OF THE FIGURES

**Figure 1** is a depiction of a typical nucleotide base arrangement of a core promoter containing the consensus sequences of TATA and INR motifs present in plant promoters. **A** designates +1 of the transcribed region.
**Figure 2** is a depiction of the complete Syn II Core Promoter Sequence with an example of a plant promoter and broth are aligned at the major start of transcription (bold letter). The TATA motif is underlined. The CaMV 35S promoter is shown with percent GC content sequences shown in parentheses.
**Figure 3** is the DNA sequence of the Rsyn7 upstream element. The TGACG motifs are indicated in bold.
**Figure 4** is a plasmid map of one embodiment of the invention comprising the Syn II Core promoter and Rsyn7 elements driving a GUS containing construct.
**Figure 5** depicts several schematics of synthetic promoters according to the present invention tested in transient and stable transformants.
**Figure 6** is a depiction of transient assay data using the plasmids incorporating the promoter sequences of the invention.
**Figure 7(A).** Rsyn7::GUS (6086) activity in T0 maize plants.
**Figure 7(B)** is a schematic of VT stage corn plants with sites of tissue samples indicated.
**Figure 8** depicts GUS activity in root segments of a segregating population of maize T1 transgenic seedlings containing the Rsyn7::GUS (6086) or the UBI::GUS (3953) construct.
**Figure 9** depicts GUS expression of three synthetic promoters in TO transgenic maize plants including the promoter sequences of the invention as comparison.

### DETAILED DESCRIPTION OF THE INVENTION

In the description that follows a number of terms are used extensively. The following definitions are provided in order to remove ambiguities in the intent or scope of their usage in the specification and claims, and to facilitate understanding of the invention.

A structural gene is a DNA sequence that is transcribed into messenger RNA (mRNA) which is then translated into a sequence of amino acids characteristic of a specific polypeptide.

A promoter is a DNA sequence that directs the transcription of a structural gene. Typically a promoter is located in the 5' region of a gene, proximal to the transcriptional start site of a structural gene.

A core promoter or minimal promoter contains the essential nucleotide sequences for proper function, including the TATA box and start of transcription. By this definition, a core promoter may or may not have detectable activity in the absence of specific sequences that may enhance the activity or confer tissue specific activity. For example, the maize core promoter consists of about 37 nucleotides 5' of the transcriptional start site of the SGB6 gene, while the Cauliflower Mosaic Virus (CaMV) 35S core promoter consists of about 33 nucleotides 5' of the transcriptional start site of the 35S genome.

ADH refers generally to a plant expressible alcohol dehydrogenase gene and specifically to the alcohol dehydrogenase gene from maize.

ADH 1 promoter refers to the DNA fragment spanning the region between nucleotide positions about -1094 to about - 106 of the alcohol dehydrogenase gene 1 from maize, or homologous fragment that is functionally equivalent. The sequence is numbered with the start of transcription site designated as +1 according to the correction published by Ellis et al. (1987) supra.

"TATA to start" shall mean the sequence between the TATA motif and the start of transcription.

A synthetic DNA is an artificially created DNA sequence that is not produced naturally, and must be introduced to an organism or to an ancestor of that organism to control or to be expressed.

OCS element refers to the TGACG motif identified from the octopine synthase gene, histone genes, enzyme genes for agropine biosynthesis, the mannopine synthase gene, the CaMV 35S gene, histone H3 gene and nopaline synthase gene. As used herein the term includes any sequence capable of binding the ASF-1 factor as identified in U.S. Patent No. 4,990,607.

An enhancer is a DNA regulatory element that can increase efficiency of transcription regardless of the distance or orientation of the enhancer relative to the start site of transcription.

The term expression refers to biosynthesis of a gene product. In the case of a structural gene, expression involves transcription of the structural gene into mRNA and then translation of the mRNA into one or more polypeptides.

A cloning vector is a DNA molecule such as a plasmid, cosmid or bacterial phage that has the capability of replicating autonomously in a host cell. Cloning vectors typically contain one or a small number of restriction endonuclease recognition sites at which foreign DNA sequences can be inserted in a determinable fashion without loss of essential biological function of the vector, as well as a marker gene that is suitable for use in the identification and selection of cells transformed with the cloning vector. Marker genes typically include genes that provide tetracycline resistance, hygromycin resistance or ampicillin resistance.

An expression vector is a DNA molecule comprising a gene that is expressed in a host cell. Typically gene expression is placed under the control of certain regulatory elements including promoters, tissue specific regulatory elements, and enhancers. Such a gene is said to be "operably linked to" the regulatory elements.

A recombinant host may be any prokaryotic or eukaryotic cell that contains either a cloning vector or an expression vector. This term also includes those prokaryotic or eukaryotic cells that have been genetically engineered to contain the cloned genes in the chromosome or genome of the host cell.

A transgenic plant is a plant having one or more plant cells that contain an expression vector.

It will be understood that there may be minor sequence variations within sequence or fragments used or disclosed in this application. These variations may be determined by standard techniques to enable those of ordinary skill in the art to manipulate and bring into utility the functional units of the promoter elements necessary to direct initiation of transcription in the structural gene followed by a plant expressible transcription termination (and perhaps polyadenylation) signal.

Plant tissue includes differentiated and undifferentiated tissues or plants, including but not limited to roots, stems, shoots, leaves, pollen, seeds, tumor tissue and various forms of cells and culture such as single cells, protoplas, embryos, and callus tissue. The plant tissue may be in planta or in organ, tissue or cell culture.

The promoter seen in SEQ ID NO:1 and/or SEQ ID NO:10, can be used to obtain high levels of expression of structural genes. The upstream element of the invention (SEQ ID NO:2) can be used in combination with other promoters or the promoter of SEQ ID NO:1 and/or SEQ ID NO:10 to potentiate levels of transcription in genetically modified plants. Production of a genetically modified plant tissue expressing a structural gene under the control of the upstream element of the invention combines teachings of the present disclosure with a variety of techniques and expedients known in the art. In most instances alternate expedients exist for each stage of the overall process. The choice of expedients depends on the variables such as the plasmid vector system chosen for the cloning and introduction of the recombinant DNA molecule, the plant species to be modified, the particular structural gene, promoter elements and upstream elements used. Persons skilled in the art are able to select and use appropriate alternatives to achieve functionality. Culture conditions for expressing desired structural genes and cultured cells are known in the art. Also as known in the art, a number of both monocotyledonous and dicotyledonous plant species are transformable and regenerable such that whole plants containing and expressing desired genes under regulatory control of the promoter molecules and upstream elements of the invention may be obtained. As is known to those of skill in the art, expression in transformed plants may be tissue specific and/or specific to certain developmental stages. Truncated promoter selection and structural gene selection are other parameters which may be optimized to achieve desired plant expression as is known to those of skill in the art and taught herein.

The selection of an appropriate expression vector will depend upon the method of introducing the expression vector into host cells. Typically an expression vector contains (1) prokaryotic DNA elements coding for a bacterial replication origin and an antibiotic resistance marker to provide for the growth and selection of the expression vector in a bacterial host; (2) DNA elements that control initiation of transcription such as a promoter; (3) DNA elements that control the processing of transcripts such as transcription termination/polyadenylation sequence; and (4) a reporter gene that is operatively linked to the DNA elements to control transcription initiation. Useful reporter genes include β-glucuronidase, β-galactosidase, chloramphenicol acetyl transferase, luciferase, green fluorescent protein (GFP) and the like. Preferably the reporter gene is either β-glucuronidase (GUS), GFP or luciferase. The general descriptions of plant expression vectors and reporter genes can be found in Gruber, et al., "Vectors for Plant Transformation, in Methods in Plant Molecular Biology & Biotechnology" in Glich et al., (Eds. pp. 89-119, CRC Press, 1993). Moreover GUS expression vectors and GUS gene cassettes are available from Clonetech Laboratories, Inc., Palo Alto, California while luciferase expression vectors and luciferase gene cassettes are available from Promega Corp. (Madison, Wisconsin).

Expression vectors containing genomic or synthetic fragments can be introduced into protoplasts or into intact tissues or isolated cells. Preferably expression vectors are introduced into intact tissue. General methods of culturing plant tissues are provided for example by Maki et al. "Procedures for Introducing Foreign DNA into Plants" in Methods in Plant Molecular Biology & Biotechnology, Glich et al. (Eds. pp. 67-88 CRC Press, 1993); and by Phillips et al. "Cell-Tissue Culture and In-Vitro Manipulation" in Corn & Corn Improvement, 3rd Edition Sprague et al. (Eds. pp. 345-387) American Society of Agronomy Inc. et al. 1988.

Methods of introducing expression vectors into plant tissue include the direct infection or co-cultivation of plant cell with *Agrobacterium tumefaciens,* Horsch et al., Science, 227:1229 (1985). Descriptions of Agrobacterium vector systems and methods for Agrobacterium-mediated gene transfer provided by Gruber, et al. supra.

Preferably, expression vectors are introduced into maize or other plant tissues using a direct gene transfer method such as microprojectile-mediated delivery, DNA injection, electroporation and the like. More preferably expression vectors are introduced into plant tissues using the microprojectile media delivery with the biolistic device.

The vectors of the invention can not only be used for expression of structural genes but may also be used in exon-trap cloning, or promoter trap procedures to detect differential gene expression in varieties of tissues, K. Lindsey et al., 1993 "Tagging Genomic Sequences That Direct Transgene Expression by Activation of a Promoter Trap in Plants", Transgenic Research 2:33-47. D. Auch & Reth, et al., "Exon Trap Cloning: Using PCR. to Rapidly Detect and Clone Exons from Genomic DNA Fragments", Nucleic Acids Research, Vol. 18, No. 22, p. 6743.

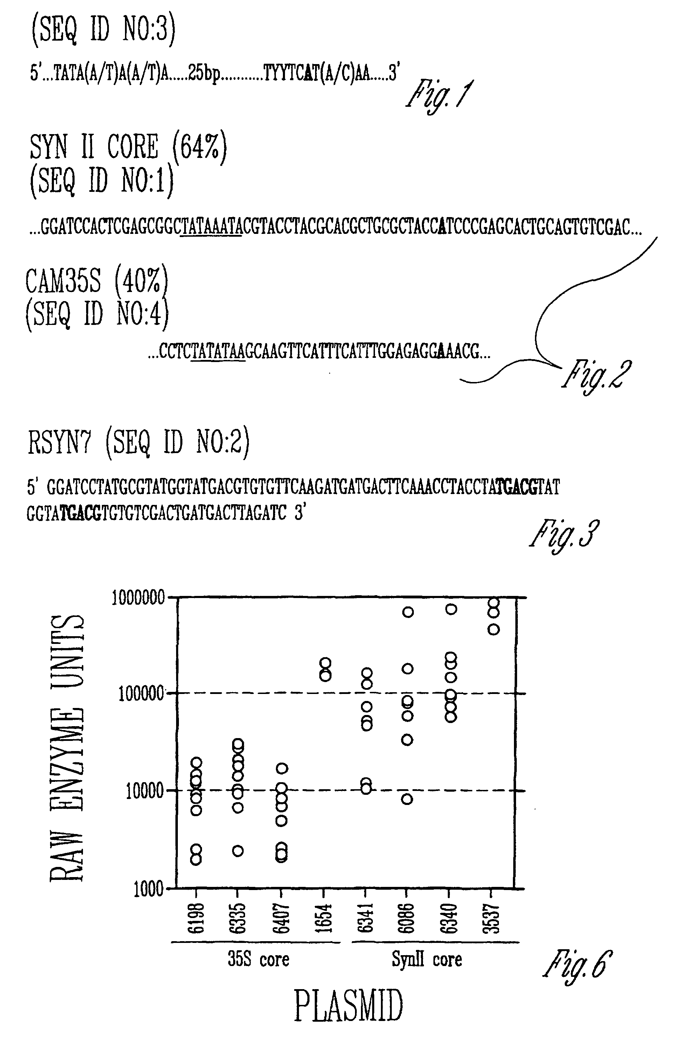

The promoter useful with the upstream element of the invention is based in part on the discovery that a GC rich "TATA to start" region in a plant promoter acts as a very strong nontissue specific core promoter inducing constitutive expression in plant cells. The TATA element of plant promoters of polII genes generally have the sequence TATA(A/T)A(A/T)A, SEQ ID NO:3 whereas the consensus of the start of transcription consists of the sequence 5'...TYYTCAT(A/C)AA...3'. SEQ ID NO:3, where the A designates the starting base for transcription. The typical plant promoter sequence is depicted in Figure 1.

Sequences intervening the TATA element and the start of transcription have been shown to play a significant role in transcriptional activation efficiency. The TATA binding protein has been shown to interact with the minor groove of the double helix binding to the TATA motif bending it towards the major groove side (Kim, et al. 1993, Nature, 365:512-520). It thus follows that sequences downstream of the TATA motif that impact this finding will affect the efficiency of stable transcriptional complex formation and ultimately expression. Surveys of the "TATA to start" regions of plant promoters show a significantly higher level of AT-rich sequences leading to the potential of minor groove compression (Yaurawaj et al Biological Abstracts Vol. 47, Issue 8, Ref. 144712, "Consensus Sequences for Plant Minimal Promoters" Annual Meeting of the American Society of Plant Physiologists, July 29-August 2, 1995, Plant Physiology 108 [2 Supp.] 1995, 114.) Generally animal promoters show a GC-rich "TATA to start" sequence that leads to a major groove compression suggesting that average plant and animal core promoter transcriptional complexes recognize and interact with a somewhat different TATA to start structure with the corresponding sequence difference. Quite surprisingly the applicant has found that a GC-rich animal type synthetic promoter works very well in plants.

While not wishing to be bound by any theory, it is possible that the AT-rich TATA motif present in a GC-rich sequence may present itself more prominently to the TATA-binding complex by a sharp demarcation of the TATA sequence from GC-rich to AT-rich. The "bounded" TATA motif would interact more tightly with the TATA-binding complex. This would improve the start of transcription efficiency, by shifting the equilibria of binding to a more stabilized form, whereas the "non-bounded" version, i.e. having more AT-Rich sequence instead of flanking the TATA motif, would potentially slide or stutter up and down and effectively reduce the efficiency of binding. Little data regarding this region of plant promoters is available except crude deletions and some point mutations. The obvious design of a synthetic core promoter for plant expression would include the AT-rich "TATA to start" sequence. However, based on the "bounded" mechanism, it is postulated by the mechanism of the invention that a more efficient core promoter would present a TATA motif imbedded in a GC-rich sequence.

Figure 2 depicts the Syn II Core promoter sequence, SEQ ID NO:1 with examples of plant core promoters aligned to the major start of transcription. Another example of a plant promoter 35S of CaMV (SEQ ID NO:4] are shown with percent GC-rich sequences shown at the right in parentheses. The Syn II Core sequence does not show any significant sequence homology to sequences in the public sequence databases.

The synthetic Syn II Core promoter sequence shows a 64% GC-rich "TATA to start" sequence different from the overall 40% GC-rich sequence present in traditional plant promoters (CaMV35S for example). The naturally occurring and isolated UBI core promoter which potentiates very high levels of activity shows a 64% GC-rich "TATA to start" sequence more similar to animal promoters. Such examples provided the impetus to design a high GC-rich "TATA to start" sequence for efficient transcription in opposition to the current dogma of plant core promoters.

Thus the synthetic plant core promoter sequence comprises a TATA motif and a "TATA to start" region that is 64% GC-rich or greater. The promoter may include restriction endo nucleotide target sites for ease of cloning. The sequence may be that of SEQ ID NO:1. As will be appreciated by those of skill in the art, several base transversions within SEQ ID NO:1 may occur which will maintain the percent GC-content and are intended within the scope of this invention. SEQ ID NO:10. For example guanines could be replaced with cytosines and vice-versa without affecting the overall efficacy of the promoter, so long as the percent GC-content is maintained.

The invention comprises a synthetic upstream element which may be positioned 5' to any naturally occurring or synthetic promoter for use in plants, particularly maize gene expression.

From the activity of numerous promoters, basic elements (binding sites) have been defined. These include for example AT-rich regions from heat shock promoters, and ASF-1 binding site (AS-1) elements present in octopine synthase (OCS) and Cauliflower Mosaic Virus promoters. AS-1 is one of the better known upstream elements and its binding sequence (OCS element) is present in many constitutive plant promoters such as the CaMV35S, *A. tumefaciens,* NOS and OCS wheat histone promoters. The OCS element was first isolated as an enhancer element in the promoter of the OCS gene where it was identified as a 16-base pair palindromic sequence (Ellis et al., 1987 MBOJ 6:11-16), but has been reduced to its essential features as a TGACG motif. See Katagiri U.S. Patent No. 4, 990, 607. The upstream element of the invention has a 71% homology to the promoter enhancer element disclosed in U.S. Patent 5,023,179 to Lam et al. The two sequences are quite different in their flanking sequences surrounding the TGACG motif, which regions have been shown to impact the level of transcription enhancement. The transcriptional enhancing activity of the OCS element correlated with the in-vitro binding of a transcriptional factor. Similar elements were also identified in the promoter regions of six other cDNA genes involved in opine synthesis and three plant viral promoters including the CaMV 35S promoter (Bouchez et al. 1989) supra. These elements were shown to bind the OCS transcription factor in-vitro and enhance transcription in plant cells.

In tobacco a DNA binding factor TGA1 was shown to interact specifically with the AS-1 element either alone or in conjunction with other promoter elements. Katagiri et al. 1989, Nature 340:727-730. This factor was also shown to be expressed in a root-preferred manner in tobacco plants. Core promoters with one or two copies of the OCS upstream element tend to potentiate gene expression whereas 4 or more repeats of this element produce more or less constitutive activity albeit low relative to intact 35S promoters.

Thus the invention incorporates a synthetic upstream element which can be used with a core promoter as disclosed herein or other core promoters to enhance gene expression. The element incorporates three OCS-like motifs and novel intervening sequences which potentiate gene expression.

Figure 3, SEQ ID NO:2 shows the complete sequence of (RSyn7) of the synthetic upstream element which incorporates at least three TGACG SEQ ID NO:5 OSC-like motifs which are indicated in bold.

Sequences flanking many elements such as the TGACG SEQ ID NO:5 motif have been shown to have profound impacts on binding affinities of DNA binding factors and thus play as an important role as the central motifs themselves. (Burrows et al. 1992, Plant Molecular Biology 19:665-675, Shindler et al. 1992, Plant Cell 4:1309-1319, Foster et al. 1994, FASEBJ 8:192-200). The novel sequences flanking the TGACG motifs in the Rsyn7 promoter have been determined and establish clear enhancement of transcriptional activity with various promoters, particularly when used with the Syn II Core promoter.

Rsyn7 upstream element has been cloned upstream of the Syn II Core promoter driving a GUS construct and has potentiated levels of GUS activity in transgenic maize plants approximately ten-fold better than the ubiquitin promoter, the strongest maize promoter to date.

The following examples are for illustration purposes only and are intended in no way to limit the scope or application of the present invention. Those of skill in the art will appreciate that many permutations can be achieved and are in fact intended to be within the scope of the invention. All reference citations throughout the specification are expressly hereby incorporated by reference.

### EXAMPLE 1

Plasmids were designed using the multiple cloning site of pBlueScriptIIKS+ from Stratagene. (To facilitate cloning of the different combination of elements). Oligonucleotides containing the sequences of the elements were synthesized with restriction endonuclease sites at the ends. Thus elements could be added or removed and replaced as needed. GUS and Luciferase were used for reporter genes.

For transient assays plasmid DNA was introduced into intact 3-day-old maize seedlings by particle bombardment. Following 16 hours incubation at 25°C in the dark, expression was assayed by measuring GUS enzyme activity in root and shoot extracts from each seedling to determine if any tissue-preferred expression was demonstrated. GUS activity was measured using a GUS-Light assay kit from Tropix (47 Wiggins Avenue, Bedford, MA 01730).

Constructs that gave high levels of expression were introduced into a cell line to produce stable transformants. These stable transformants (TO) were assayed by PCR to determine the presence of the GUS gene by MUG (4-methylumbelliferyl-glucuronide) assay to quantify the activity level of the GUS protein being produced. When the plants were ready to be transferred to the greenhouse they were assayed histochemically with X-gluc to determine where the GUS product was being synthesized. Plants demonstrating preferred expression levels were grown in the greenhouse to V6 stage.

### EXAMPLE 2

### Construction of Plasmids Containing the Syn II Core Promoter

Standard molecular biological techniques were carried out according to Maniantis et al. (1982) Molecular Cloning: A Laboratory Manual, Cold Spring Harbor Laboratory, Cold Spring Harbor, N.Y. All plasmids utilized in the invention can be prepared according to the directions of the specification by a person of ordinary skill in the art without undue experimentation employing materials readily available in the art.

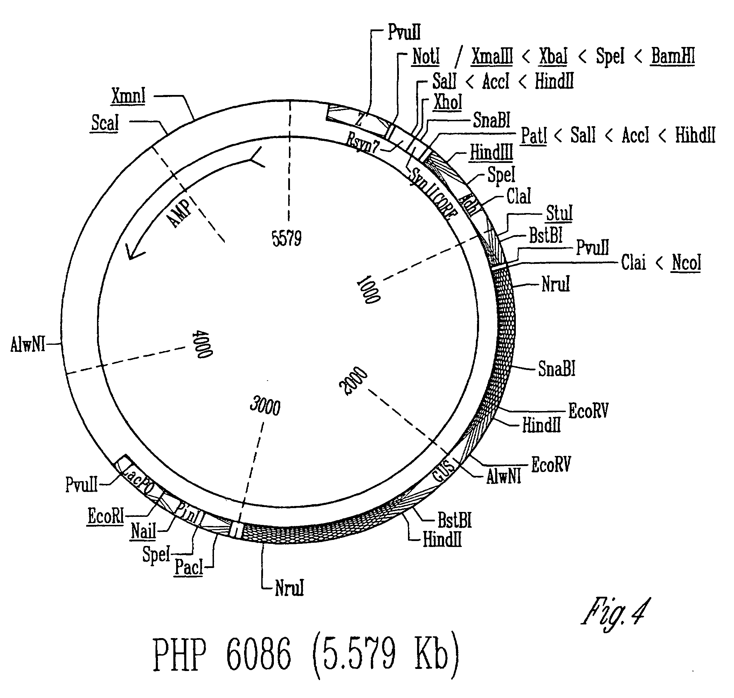

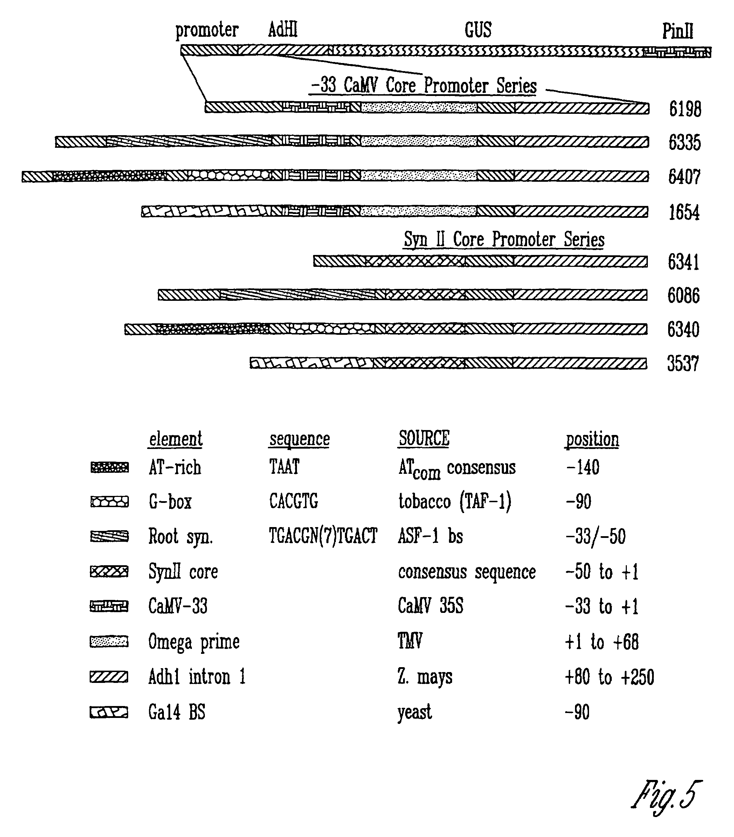

Oligos N306 SEQ ID NO:6 TCGACACTGC AGCTCTAGGG ATGGTAGCGC AGGGTGCGTA GGTACGTATT TATAGCCGCT CGAGTG-3' and N307 SEQ ID NO:7 GATCCACTCG AGCGGCTATA AATACGTACC TACGCACCCT GCGCTACCAT CCCTAGAGCT GCAGTG-3' were synthesized according to directions on an automated DNA synthesizer (such as Applied Biosystems Inc. DNA Synthesizer (Model 380B). These automated synthesizers are commercially available. The oligos were then ligated to the BamHI fragment of the pBlueScriptIIKS+ plasmid comprising of the β-glucuronidase gene interrupted by the maize ADH1 intron 1 intronic region. A map of a plasmid incorporating both the Syn II Core promoter and the upstream element is disclosed as Figure 4. Several other embodiments are shown in other plasmids depicted in Figure 5. Plasmid numbers are shown to the right of each promoter diagram with the corresponding legend placed below the diagrams. The top diagram shows the complete transplant transcriptional unit with the subsequent diagrams focusing on the salient differences between 35S and Syn II Core promoters. The legend shows the number and nature of the various promoter subelements, the sequence if relatively short, the source of the element and position relative to the start of transcription.

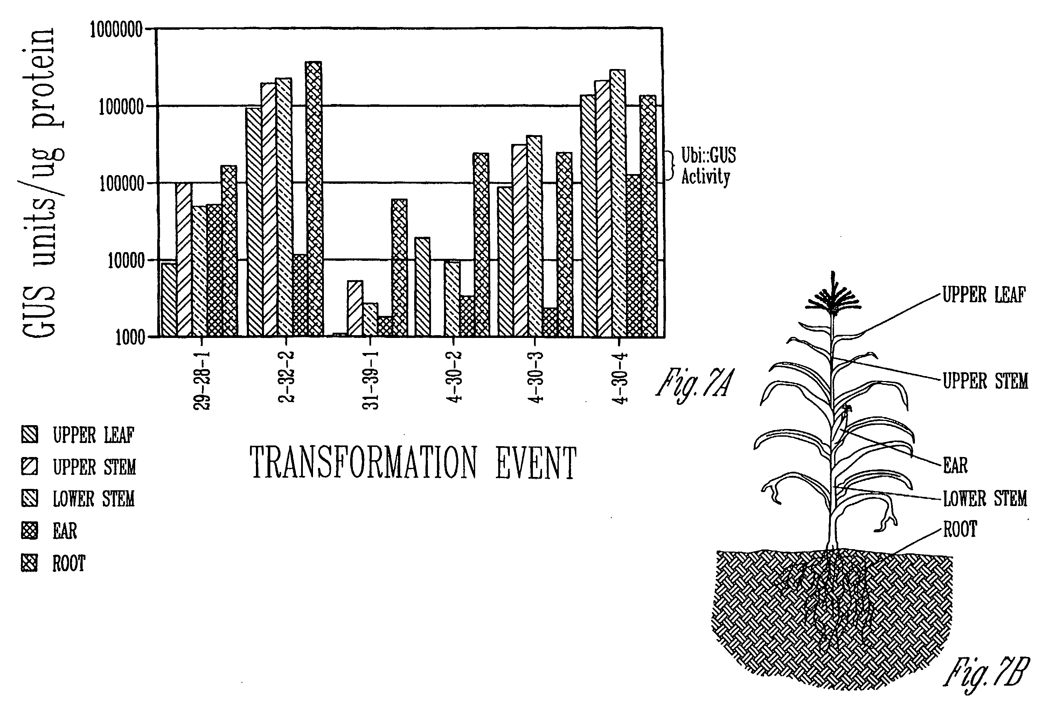

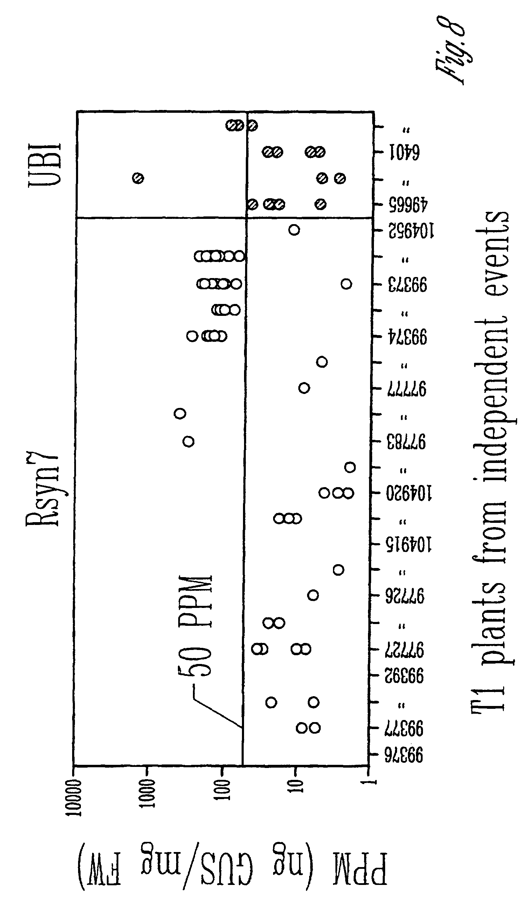

The sequence of the core promoter consists of 35 base pairs with enzyme sites upstream of a TATA box and a start of transcription with 10 to 15 base pairs downstream. Upstream elements (Gal 4-binding sites, Rsyn, AT-GBL etc.) were fused to the core sequence with ADHI-intron and different marker genes (LUC or GUS) and were demonstrated functional both in transient assays (Figure 6) and Rsyn stably transformed plants (Figure 7).

### EXAMPLE 3

### Construction of Upstream Element Rsyn7 Fused to Syn II Core Promoter Resulting in Plasmids P5903 and P6086

Oligos for constructing the Rsyn7 promoter subelement N1965: (SEQ ID NO:8) GATCCTATGA CGTATGGTAT GACGTGTGTT CAAGATGATG ACTTCAAACC TACCTATGAC GTATGGTATG ACGTGTGTCG ACTGATGACT TA and N1966: (SEQ ID NO:9) GATCTAAGTC ATCAGTCGAC ACACGTCATA CCATACGTCA TAGGTAGGTT TGAAGTCATC ATCTTGAACA CACGTCATAC CATACGTCA TAG were synthesized as earlier described and cloned into the pBlueScriptIIKS+ plasmid BamH1 site. The oligos were annealed and cloned into a P3398 plasmid upstream of the Syn II Core sequence and resulted in several versions of the original Rsyn7 sequence due to spontaneous deletions. The Rsyn7-2 version involved a single base deletion resulting in a 3X reiterative TGACG motif upstream of the Syn II Core promoter (Rsyn7::LUC, P5903). The LUC coding sequence was replaced by GUS coding sequence to produce the Rsyn7 GUS construct P6086. P6086 was later introduced into transgenic maize resulting in high levels of constitutive activity in four of the six active events examined (Figure 7).

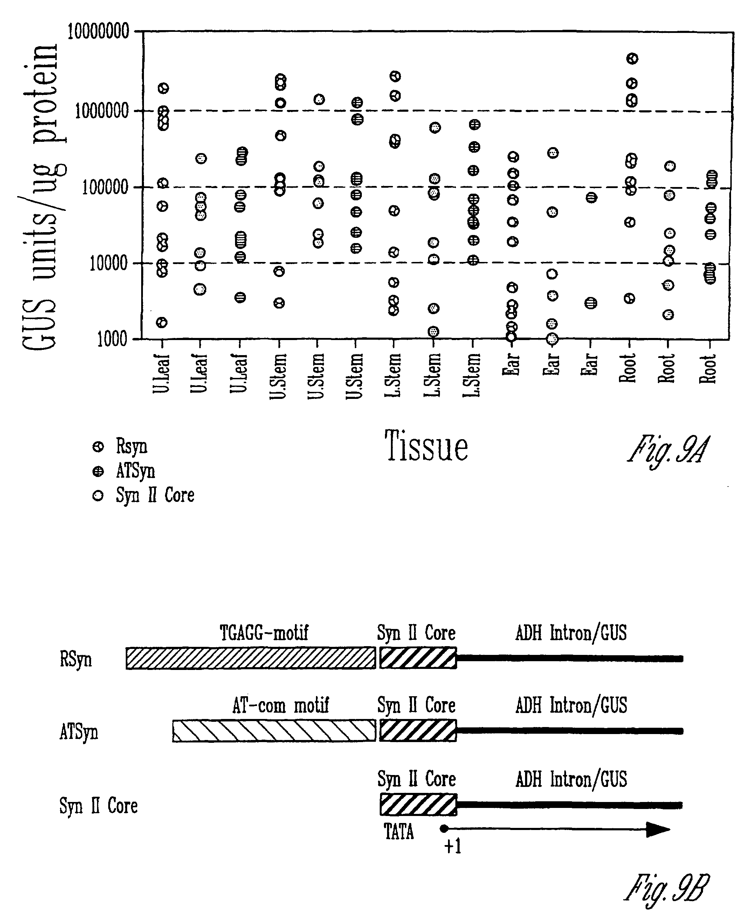

The progeny from T0 plants from several transformation events were examined and GUS activity ranging from 1 to 400 PPM (micrograms GUS enzyme/GFW in root tissue of 7-day old seedlings) Figure 8. These T0 and T1 plants generally produced 4X-10X greater GUS activity than plants harboring the ubiquitin::GUS reporter gene.

Thus from the foregoing, it can be seen that the invention accomplishes at least all of its objectives.

### EXAMPLE 4

### Transformation and Expression with Syn II Core Promoter and/or Rsyn7 Upstream Element

Using transient bombardment assays the Syn II Core promoter sequence was compared against the 35S core sequence either alone or in conjunction with numerous activation elements. Figure 6 is a depiction of transient assay data using the plasmids incorporating the promoter sequences of the invention and shows transient GUS or LUC activity in three-day old maize roots or BMS callus bombarded with chimeric promoter::GUS or LUC constructs. The -33 CaMV35S in the Syn II Core promoter versions of the synthetic promoter::GUS (or LUC) constructs were bombarded into three-day old maize roots (or cultured BMS calli as described hereinafter) and assayed for enzyme activity 20 hours after bombardments. The data shown are the raw enzyme units of a compilation of at least three experiments and have not been normalized in any fashion due to the inherent variability of the transient assays. Control plasmids 1654 and 3537 are the LUC constructs tested in maize bms calli. There is approximately 4 to 20 fold difference in transient activity between the 35S and Syn II Core versions. The Y axis is in log scale. Both core promoters were driving a GUS containing construct (Figures 4 and 5) and generated a basal level of activity (Figure 6). However when activator elements were placed upstream of the TATA motif, the Syn II Core provided generally higher levels of activity (2-4 fold better) in corn cells than when the activator elements were placed upstream of the 35S core (Figure 6).

The Syn II Core sequence has been shown to potentiate activity in stably transformed plants. Further with certain activator sequences upstream of the TATA element activity levels in stably transformed corn plants reached levels ten-fold greater than maize ubiquitin constructs which produces extremely high levels of activity.

Figures 7 and 8 show GUS activity levels from isolated tissues of VT stage T0 plants and root tissue from T1 seedlings, respectively. These data demonstrate that this core sequence can participate in potentiating very high levels of activity as a functional partner for the active chimeric promoters. Figure 7 shows the Rsyn7::GUS (6086) activity in T0 maize plants. VT stage plants with ears post pollinated 3 to 8 days were dissected and assayed for GUS activity. 7A depicts GUS expression in designated tissues. 7B depicts a schematic of corn plants with sites of measurement indicated. Plants from T0 events that demonstrated a range of activities with the Rsyn7 promoter were assayed. Log scale again noted. The activity range for UBI::GUS plants is indicated at the right of graph for comparisons. These data demonstrate that the Rsyn7 promoter can potentiate activity to ten-fold above levels of the ubiquitin promoter yet shows little tissue preference making the Rsyn7 suitable as a strong constitutive promoter.

Figure 8 depicts GUS activity in root segments of a segregating population of maize T1 transgenic seedlings containing the Rsyn7::GUS (6086) or the UBI::GUS (3953) construct. 1 cm root segments from six to seven-day old transgenic maize seedlings were dissected, weighed and assayed for GUS using GUS-light kit. Activity is represented as parts per million of fresh weight. The root activity of several T1 plants harboring the Rsyn7::GUS promoter shows higher activity than much of the activity levels produced by the UBI promoter. This is consistent with data from T0 transgenic plant. Activity levels in Rsyn7::GUS containing young leaves are also much higher than the activity levels of UBI::GUS-containing young leaves (data not shown). The Syn II Core sequence was shown to function well with a variety of upstream elements including GAL 4 binding sites, Rsyn7 elements, GBL elements, etc.

Figure 9 shows GUS expression of three synthetic promoters in TO transgenic maize plants. Dissected tissues (See Figure 7B) from VT stage transgenic TO plants harboring Rsyn7 (Rsyn), Atsyn or the Syn II Core (syn-core) promoter::GUS constructs were quantitatively assayed for GUS activity. Each circle represents an average of tissue activity of transgenic maize plants from a single transformation event. The TGACG-motif corresponds to the Rsyn7 sequence and the "At-com" motif refers to the consensus AT-like composite element, Atcom, from W. Gurley, et al. 1993. *In*: Control of Plant Gene Expression. ed. by Desh Pal Verma. CRC press, Boca Raton, FL. pp. 103-123. Syn-core refers to the Syn II Core promoter sequence containing the TATA element and the start of transcription.

## Claims

1. A synthetic upstream element having a sequence of SEQ ID NO:2.

2. An expression cassette comprising:
a promoter;
a structural gene operatively linked to said promoter sequence;
a polyadenylation signal; and
a synthetic upstream element comprising SEQ ID NO:2 operatively linked to said promoter so that expression is enhanced.

3. An expression cassette as claimed in claim 2, wherein the promoter is a synthetic promoter comprising a TATA motif; a transcription start site, and a region between the TATA motif and the transcription start site that is at least 64% GC rich.

4. The expression cassette of claim 3, wherein said promoter is SEQ ID NO:1 or SEQ ID NO:10.

5. A nucleic acid vector comprising the expression cassette of claim 2, 3 or 4.

6. A nucleic acid vector comprising
(i) a promoter operatively linked to a structural gene, wherein the promoter is a synthetic DNA plant promoter sequence which comprises:
a TATA motif;
a transcription start site; and
a region between the TATA motif and the start site that is at least 64% GC-rich; and
(ii) an upstream element operatively linked to said promoter comprising the sequence of SEQ ID NO:2.

7. The vector of claim 6, wherein said promoter sequence is SEQ ID NO:10 or SEQ ID NO:1.

8. The vector of claim 6 or claim 7, wherein said vector is a cloning vector.

9. The vector of claim 6 or claim 7, wherein said vector is an expression vector.

10. The vector of any one of claims 6 to 9, further comprising a marker gene for selection of transformed cells.

11. The vector of claim 10, wherein said marker gene is an antibiotic resistance gene.

12. The vector of any one of claims 6 to 11, further comprising a polyadenylation signal.

13. A prokaryotic or eukaryotic host cell transformed with the nucleic acid vector of any one of claims 5 to 12.

14. A transgenic plant comprising a plant cell or ancestor thereof which has been transformed with the vector of any one of claims 5 to 12.

15. Transformed seed of the transgenic plant as claimed in claim 14.

## Patentansprüche

1. Synthetisches stromaufwärts gelegenes Element mit einer Sequenz der SEQ ID NR. 2.

2. Expressionskassette, aufweisend:
einen Promotor;
ein Strukturgen, das mit der Promotorsequenz betriebsbereit verknüpft ist;
ein Polyadenylierungssignal; und
ein synthetisches stromaufwärts gelegenes Element, aufweisend die SEQ ID NR. 2, das mit dem Promotor betriebsbereit verknüpft ist, so dass die Expression verstärkt wird.

3. Expressionskassette nach Anspruch 2, wobei der Promotor ein synthetischer Promotor ist, der ein TATA-Motif, eine Transskriptions-Startstelle und eine Region zwischen dem TATA-Motif und der Transskriptions-Startstelle, die zu mindestens 64 % GC-reich ist, aufweist.

4. Expressionskassette nach Anspruch 3, wobei der Promotor die SEQ ID NR. 1 oder die SEQ ID NR. 10 ist.

5. Nucleinsäurevektor, der die Expressionskassette nach Anspruch 2, 3 oder 4 aufweist.

6. Nucleinsäurevektor, aufweisend:
(i) einen mit einem Strukturgen betriebsbereit verknüpften Promotor, wobei der Promotor eine synthetische pflanzliche DNA-Promotorsequenz ist, die aufweist:
ein TATA-Motif;
eine Transkriptionsstartstelle; und
eine Region zwischen dem TATAMotif und der Startstelle, die zu mindestens 64 % GC-reich ist; und
(ii) ein stromaufwärts gelegenes Element, das mit dem Promotor betriebsbereit verknüpft ist, welches die SEQ ID NR. 2 aufweist.

7. Vektor nach Anspruch 6, wobei die Promotorsequenz die SEQ ID NR. 10 oder die SEQ ID NR. 1 ist.

8. Vektor nach Anspruch 6 oder Anspruch 7, wobei der Vektor ein Klonierungsvektor ist.

9. Vektor nach Anspruch 6 oder Anspruch 7, wobei der Vektor ein Expressionsvektor ist.

10. Vektor nach einem der Ansprüche 6 bis 9, der weiterhin ein Markergen zur Selektion transformierter Zellen aufweist.

11. Vektor nach Anspruch 10, wobei das Markergen ein Antibiotikaresistenzgen ist.

12. Vektor nach einem der Ansprüche 6 bis 11, der weiterhin ein Polyadenylierungssignal aufweist.

13. Prokariotische oder eukariotische Wirtszelle, die mit dem Nukleinsäurevektor nach einem der Ansprüche 5 bis 12 transformiert worden ist.

14. Transgene Pflanze, aufweisend eine pflanzliche Zelle oder eine Vorfahrin davon, die mit dem Vektor nach einem der Ansprüche 5 bis 12 transformiert worden ist.

15. Transformierter Samen der transgenen Pflanze nach Anspruch 14.

## Revendications

1. Elément synthétique en amont présentant une séquence de la SEQ ID NO:2.

2. Cassette d'expression comprenant :
un promoteur;
un gène de structure lié de manière opérationnelle à ladite séquence de promoteur;
un signal de polyadénylation; et
un élément synthétique en amont comprenant la SEQ ID NO:2 lié de manière opérationnelle audit promoteur de sorte à augmenter l'expression.

3. Cassette d'expression comme revendiquée à la revendication 2, dans laquelle le promoteur est un promoteur synthétique comprenant un motif TATA; un site d'initiation de la transcription, et une région entre le motif TATA et le site d'initiation de la transcription qui est riche en GC au moins à 64%.

4. Cassette d'expression de la revendication 3, dans laquelle ledit promoteur est la SEQ ID NO:1 ou la SEQ ID NO:10.

5. Vecteur d'acides nucléiques comprenant la cassette d'expression de la revendication 2, 3 ou 4.

6. Vecteur d'acides nucléiques comprenant
(i) un promoteur lié de manière opérationnelle à un gène de structure, dans lequel le promoteur est une séquence synthétique de promoteur végétal d'ADN qui comprend :
un motif TATA;
un site d'initiation de la transcription; et
une région entre le motif TATA et le site d'initiation de la transcription qui est riche en GC au moins à 64%; et
(ii) un élément en amont lié de manière opérationnelle audit promoteur comprenant la séquence de la SEQ ID NO:2.

7. Vecteur de la revendication 6, dans lequel ladite séquence de promoteur est la SEQ ID NO:10 ou la SEQ ID NO:1.

8. Vecteur de la revendication 6 ou de la revendication 7, dans lequel ledit vecteur est un vecteur de clonage.

9. Vecteur de la revendication 6 ou de la revendication 7, dans lequel ledit vecteur est un vecteur d'expression.

10. Vecteur de l'une quelconque des revendications 6 à 9, comprenant en outre un gène marqueur pour une sélection de cellules transformées.

11. Vecteur de la revendication 10, dans lequel ledit gène marqueur est un gène de résistance à un antibiotique.

12. Vecteur de l'une quelconque des revendications 6 à 11, comprenant en outre un signal de polyadénylation.

13. Cellule hôte de procaryote ou d'eucaryote transformée avec le vecteur d'acides nucléiques de l'une quelconque des revendications 5 à 12.

14. Plante transgénique comprenant une cellule végétale ou un ancêtre de celle-ci que l'on a transformée avec le vecteur de l'une quelconque des revendications 5 à 12.

15. Graine transformée de la plante transgénique comme revendiquée à la revendication 14.


### SEQUENCE LISTING

(1) GENERAL INFORMATION:
   (i) APPLICANT: Pioneer Hi-Bred International, Inc.
   (ii) TITLE OF INVENTION: Synthetic Plant Core Promoter and Upstream Regulatory Element
   (iii) NUMBER OF SEQUENCES: 10
   (iv) CORRESPONDENCE ADDRESS:
      (A) ADDRESSEE: Pioneer Hi-Bred International, Inc.
      (B) STREET: 7100 N.W. 62nd Avenue, Darwin Building
      (C) CITY: Johnston
      (D) STATE: Iowa
      (E) COUNTRY: United States
      (F) ZIP: 50131
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: JERVIS, Herbert H.; SWEENEY, Patricia A.; BOBROWICZ, Donna; RAN, David.
      (B) REGISTRATION NUMBER:
   (ix) TELECOMMUNICATION INFORMATION:
      (A) TELEPHONE: 515-334-4468
      (B) TELEFAX: 515-334-6883
(2) INFORMATION FOR SEQ ID NO:1:
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:1:
   GGATCCACTC GAGCGGCTAT AAATACGTAC CTACGCACGC TGCGCTACCA TCCCGAGCAC 60
   TGCAGTGTCG AC 72
(2) INFORMATION FOR SEQ ID NO:2:
   (i) SEQUENCE CHARACTERISTICS:
      (A) LENGTH: 96 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:2:
   GGATCCTATG CGTATGGTAT GACGTGTGTT CAAGATGATG ACTTCAAACC TACCTATGAC 60
   GTATGGTATG ACGTGTGTCG ACTGATGACT TAGATC 96
(2) INFORMATION FOR SEQ ID NO:3:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:3:
   TATAWAWATY YTCATMAA 18
(2) INFORMATION FOR SEQ ID NO:4:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:4:
   CCTCTATATA AGCAAGTTCA TTTCATTTGG AGAGGAAACG 40
(2) INFORMATION FOR SEQ ID NO:5:
   (i) SEQUENCE CHARACTERISTICS:
      (A) LENGTH: 5 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:5:
   TGAGC 5
(2) INFORMATION FOR SEQ ID NO:6:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:6:
   TCGACACTGC AGCTCTAGGG ATGGTAGCGC AGGGTGCGTA GGTACGTATT TATAGCCGCT 60
   CGAGTG 66
(2) INFORMATION FOR SEQ ID NO:7:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:7:
   GATCCACTCG AGCGGCTATA AATACGTACC TACGCACCCT GCGCTACCAT CCCTAGAGCT 60
   GCAGTG 66
(2) INFORMATION FOR SEQ ID NO:8:
   (i) SEQUENCE CHARACTERISTICS:
      (A) LENGTH: 92 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:8:
   GATCCTATGA CGTATGGTAT GACGTGTGTT CAAGATGATG ACTTCAAACC TACCTATGAC 60
   GTATGGTATG ACGTGTGTCG ACTGATGACT TA 92
(2) INFORMATION FOR SEQ ID NO:9:
   (i) SEQUENCE CHARACTERISTICS:
      (A) LENGTH: 92 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:9:
   GATCTAAGTC ATCAGTCGAC ACACGTCATA CCATACGTCA TAGGTAGGTT TGAAGTCATC 60
   ATCTTGAACA CACGTCATAC CATACGTCAT AG 92
(2) INFORMATION FOR SEQ ID NO:10:
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:10:
   GGATCCACTC GAGCGGCTAT AAATASSTAS STASSSASSS TSSSSTASSA TCCCGAGCAC      60
   TGCAGTGTCG AC                                                          72